(19) Europäisches Patentamt
European Patent Office
Office européen des brevets

(11) Publication number: 0 450 527 A2

# (12) EUROPEAN PATENT APPLICATION

(21) Application number: 91105051.6

(22) Date of filing: 28.03.91

(51) Int. Cl.5: C07C 233/18, C07C 231/02, C07D 263/14, A61K 7/06, A61K 7/48

(30) Priority: 30.03.90 JP 85224/90
25.04.90 JP 109762/90

(43) Date of publication of application:
09.10.91 Bulletin 91/41

(84) Designated Contracting States:
AT CH DE ES FR GB LI NL

(71) Applicant: KAO CORPORATION
1-14-10, Nihonbashikayaba-cho, Chuo-ku
Tokyo(JP)

(72) Inventor: Nakagawa, Shoji
No. 1130, Nishihama
Wakayama-shi, Wakayama(JP)
Inventor: Kojima, Masayo
5-28-403, Nishi Takamatsu 1-chome
Wakayama-shi, Wakayama(JP)
Inventor: Yokota, Yukinaga
208-10, Tottorinaka, Hannancho
Sennan-gun, Osaka(JP)
Inventor: Yahagi, Kazuyuki
1-6-749, Ojima 6-chome
Koto-ku, Tokyo(JP)
Inventor: Tashiro, Kazuhiro
20-1, Innai 3-chome
Funabashi-shi, Chiba(JP)

(74) Representative: Hansen, Bernd, Dr. Dipl.-Chem. et al
Hoffmann, Eitle & Partner Patent- und Rechtsanwälte Arabellastrasse 4 Postfach 81 04 20
W-8000 München 81(DE)

## (54) N-Tris(hydroxymethyl)methylfatty acid amides and cosmetic compositions containing same.

(57) An N-tris(hydroxymethyl)methyl-fatty acid amide of formula (I):

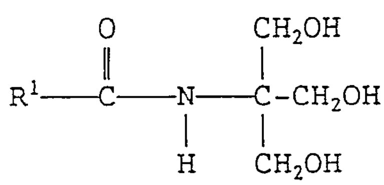

(I)

where $R^1$ is a branched alkyl group containing 4 to 27 carbon atoms, and a method for producing the fatty acid amide are disclosed. A cosmetic composition containing the novel amide and other amides, and a method of moisturizing a cosmetic composition using the novel amide and other amides are also disclosed.

EP 0 450 527 A2

## FIELD OF THE INVENTION

This invention relates to novel fatty acid amides and a novel cosmetic composition containing the same. More particularly, the present invention relates to novel fatty acid amides which are useful as bases, emulsifiers, lubricants and the like in a cosmetic preparation to be applied to the hair or skin, to a method for producing the amides, to an intermediate in the production of the amide and to a novel cosmetic composition containing the fatty acid amides, which have good emulsion stability and, when applied, show good spreadability or extendibility with a favorable feeling during use without stickiness.

## BACKGROUND OF THE INVENTION

Various humectants have been used in cosmetics, however, they have problems. For instance, when used in small amounts, they produce their humectant (moisture-retaining) effect only to an unsatisfactory extent while, when used in large amounts, they impair the feel and/or stability of the resulting systems.

Emulsion-type cosmetics are in wide use since, owing to their composition, they can provide the skin or hair with appropriate amounts of oil and moisture. Various emulsions differing in physical properties and feeling during use can be obtained by varying the oil phase components and the contents thereof.

However, emulsions are thermodynamically unstable and can not be stabilized satisfactorily. A large number of studies and attempts have been directed to the stabilization of emulsions. One of the efficient measures hitherto known is the addition of an emulsification aid or promoter, which may be cationic, anionic, amphoteric or nonionic. Nonionic emulsification aids are effective for a wide variety of oil phase components, hence are most preferred.

As an example of the addition of nonionic emulsification aide, higher alcohols, such as cetanol and cetostearyl alcohol, are added to emulsions for cosmetic use and it is known that such higher alcohols have liquid crystal structures in the emulsions and thereby improve the stability thereof. The addition of higher alcohols, however, has its drawbacks although it is effective in preventing high-melting fats and oils from crystallizing. When higher alcohols are used in amounts sufficient to maintain emulsions in a stable state, the emulsions acquire a very high viscosity and become not only poor in spreadability on application but also highly greasy and sticky.

Furthermore, the liquid crystal structures of higher alcohols have the problem that destruction of the structures results in precipitation of crystals having a pearl-like luster and in viscosity decrease, among others.

Therefore, compositions containing a nonionic emulsification aid capable of forming a lamellar liquid crystal structure have been developed. For instance, JP-B-38-5050 (the term "JP-B" as used herein means "examined Japanese Patent Publication") discloses skin care or hair care compositions containing a triol of the formula:

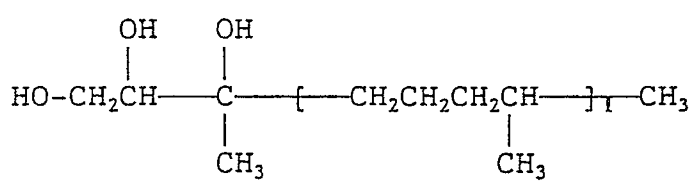

wherein ℓ represents an integer of 1 to 3. However, this triol turns into an inverted middle liquid crystal in aqueous solution. It gives a strong greasy feeling and is insufficient in humectancy-securing effect.

JP-A-63-23737 (the term "JP-A" as used herein means "unexamined published Japanese Patent Application") (corresponding to GB-A-2189457) discloses an aqueous dispersion of niosomes comprising lipid lamellae of a polyglycerol ether used as a nonionic amphiphilic substance, with a water-soluble active substance being included in the aqueous section between the lipid layers. The stability of the active substance is thus improved. However, for the formation of niosomes containing the active substance in their vesicle, treatment at a high temperature not lower than 80 °C is required, making it difficult to apply this technique to active substances having poor high-temperature stability.

JP-B-58-8287 (corresponding to GB-A-1539625), JP-B-61-56016 (corresponding to GB-A-1539625), JP-A-57-77613 (corresponding to EP-A-43827) and JP-A-57-94326 (corresponding to EP-A-43327) also disclose aqueous dispersions of lipid spheres in which a polyglycerol ether is used. However, the processes for manufacturing such dispersions require homogenization or include dissolution of a lipid in an organic

solvent, such as chloroform-methanol, and the subsequent evaporation of the solvent and are complicated and difficult to carry out on a commercial scale.

As mentioned above, the prior art nonionic emulsification aids are disadvantageous in that they form liquid crystals only in a narrow concentration range and at a relatively high temperature and that commercial scale liquid crystal formation using them is difficult to attain.

Generally, cosmetic compositions containing high-melting active substances that are not uniformly emulsified or dispersed but are in a separated state do not produce a satisfactory humectancy-securing effect. Some of the nonionic aids can serve as humectants but their effect is unsatisfactory.

Accordingly, cosmetic compositions which can remain homogeneous and stable at use temperatures and in a wide emulsification aid concentration range without undergoing changes such as crystal form transition and at the same time have a good moisture-retaining effect are desired.

It is known that N-tris(hydroxymethyl)methyl-fatty acid amides can be used as auxiliary detergent components (United States Patent No. 2,927,081). However, whether they have excellent moisture-retaining activity has remained unknown so far.

On the other hand, trimethylol compounds such as trimethylolpropane, trimethylolethane and trimethylolnonane, are used as raw materials for the production of alkyd resins, polyurethanes and polyesters, among others. These known trimethylol compounds are also used as bases, emulsifiers and lubricants for cosmetic preparations to be applied to the hair and skin, for instance. For cosmetic purposes however, they are not fully satisfactory when evaluated from the performance characteristics viewpoint; for instance, some are high-melting solids, some are difficult to uniformly disperse in water because of their inadequate hydrophile-lipophile balance, and some have poor compatibility with solvents.

Accordingly, there is a need for novel trimethylol compounds having characteristics favorable for use as a base, an emulsifier, a lubricant or the like for cosmetics to be applied to the hair or skin.

## SUMMARY OF THE INVENTION

Under the circumstances, the present inventors have made intensive investigations in an attempt to solve the problems mentioned above. As a result, they found that certain N-tris(hydroxymethyl)methyl-fatty acid amides have those characteristics that are required of cosmetic bases, emulsifiers and lubricants, and that these fatty acid amides can be synthesized in high purity and high yield from inexpensive and readily available, materials.

Further, they found that these fatty acid amides have excellent humectant activity, that cosmetic excellent in humectancy can be obtained using these fatty acid amides, and that when incorporated in emulsion-type cosmetic compositions, the amides contribute not only to good humectancy but also to the stabilization of the emulsions. The amides extend the homogeneity range of high melting active ingredients and provide cosmetics capable of spreading well upon application, giving a refreshing feeling during use without stickiness. The present invention has been completed based on these findings.

Accordingly, the present invention provides an N-tris(hydroxymethyl)methyl-fatty acid amides of formula (I):

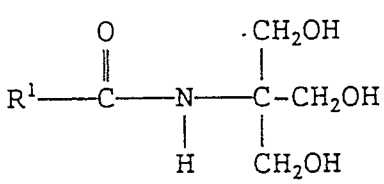

(I)

wherein $R^1$ represents a branched alkyl group containing 4 to 27 carbon atoms.

The present invention further provides a method for producing the fatty acid amides of formula (I) given above, which comprises reacting a branched fatty acid of formula (II):

$R^1COOH$ (II)

wherein $R^1$ is as defined above, with tris(hydroxymethyl)aminomethane of formula (III):

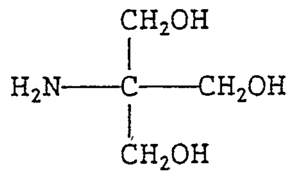

(III)

and hydrolyzing the resulting compound of formula (IV):

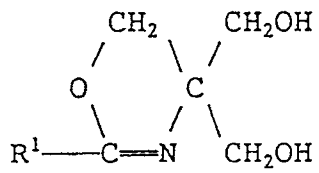

(IV)

wherein $R^1$ is as defined above, in a mixed solvent composed of a lower alcohol containing 1 to 6 carbon atoms and water.

The present invention furthermore provides an intermediate in the production of the N-tris-(hydroxymethyl)methyl-fatty acid amide of formula (I) above.

In a further aspect, the invention provides, as preferred species, branched fatty acid amides of formula (Ia):

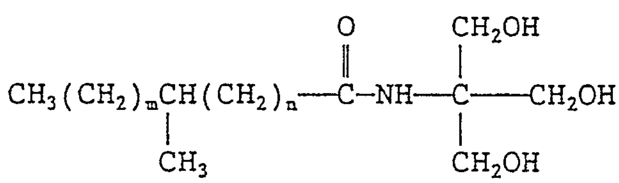

(Ia)

wherein m and n each independently represents an integer of 0 to 20 provided that the sum of m and n is 1 to 20.

The present invention also provides a cosmetic composition and a humectant each containing an N-tris-(hydroxymethyl)methyl-fatty acid amide of formula (I'):

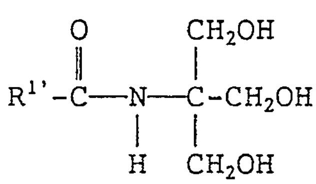

(I')

wherein $R^{1'}$ represents a branched alkyl or linear alkenyl group each containing 4 to 27 carbon atoms.

## BRIEF DESCRIPTION OF THE DRAWINGS

Fig. 1 shows an infrared absorption spectrum of N-tris(hydroxymethyl)methylisostearamide obtained in Example 1;

Fig. 2 shows a nuclear magnetic resonance spectrum of the same; and

Fig. 3 is a diagram showing the time course of water retention (changes in water content) for each of the samples tested in Example 3.

## DETAILED DESCRIPTION OF THE INVENTION

The branched alkyl group represented by $R^1$ in formula (I) above contains 4 to 27 carbon atoms, preferably 10 to 22 carbon atoms, and more preferably 14 to 20 carbon atoms.

The N-tris(hydroxymethyl)methyl-fatty acid amides of the present invention is represented by the above formula (I). As typical examples of the group

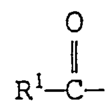

in formula (I), there may be mentioned branched-chain acyl groups such as isostearoyl, 2-octyldecanoyl and 2-heptylundecanoyl groups. Particularly preferred are those branched acyl groups that are represented by formula (V) or (VI) given below.

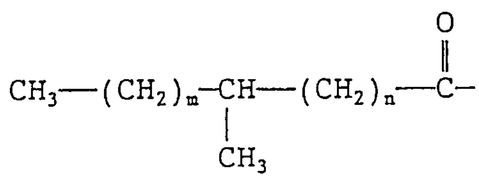 (V)

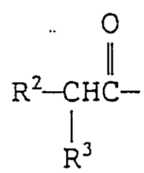 (VI)

In formula (V), m and n each represent an integer of 0 to 20, preferably 0 to 16, provided that the sum of m and n is 1 to 20, preferably 10 to 16. In formula (VI), $R^2$ and $R^3$ each represent a linear alkyl group containing 2 to 24 carbon atoms, provided that the sum of the carbon numbers of the alkyl groups represented by $R^2$ and $R^3$ is 4 to 26. Typical examples of these branched acyl groups include residues of carboxylic acids which are obtained by radical addition of an $\alpha$-olefin at $\alpha$-position of a carboxyl group or a Guerbet acid.

More preferred as

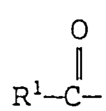

are those groups of formula (V) in which the sum of m and n is 10 to 16. Among them, isostearoyl (wherein m + n = 14) and the group of formula (V) in which m is 1 to 24 and n is 0 are most preferred. Those acyl groups which have the branching methyl group approximately in the middle of their principal chain are further preferred.

The N-tris(hydroxymethyl)methyl-fatty acid amides (I) whose

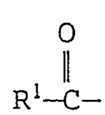

group is represented by the above formula (V) occur as lamellar liquid crystals in the presence of water at room temperature and are characterized by their good compatibility with most solvents and by their ability to disperse almost uniformly in water upon admixing with water.

In accordance with the invention, the N-tris(hydroxymethyl)methyl-fatty acid amides (I) can be produced

advantageously by the following process.

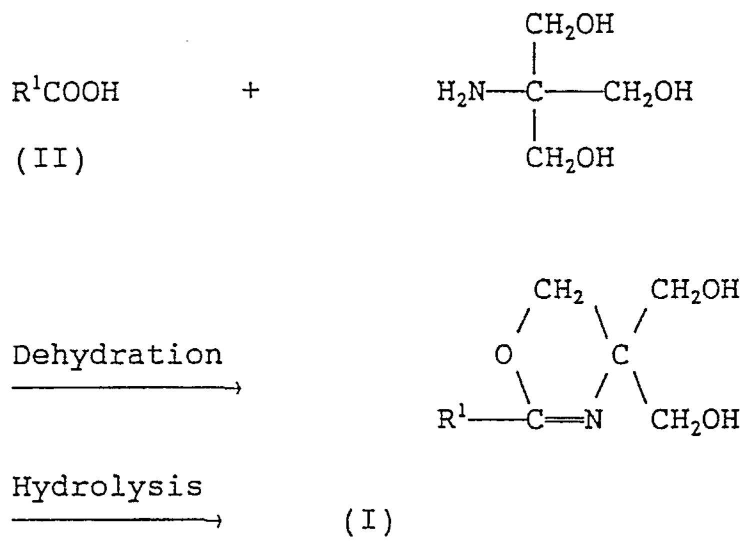


(II) (III) (IV) (I)

In formulas (II) and (IV), $R^1$ is as defined above.

Thus, a fatty acid (II) is reacted with tris(hydroxymethyl)aminomethane (III) and the resulting compound (IV) is then hydrolyzed to give the corresponding compound (I).

For the preferred compounds having an acyl group of formula (Ia), the above process may be illustrated as follows:

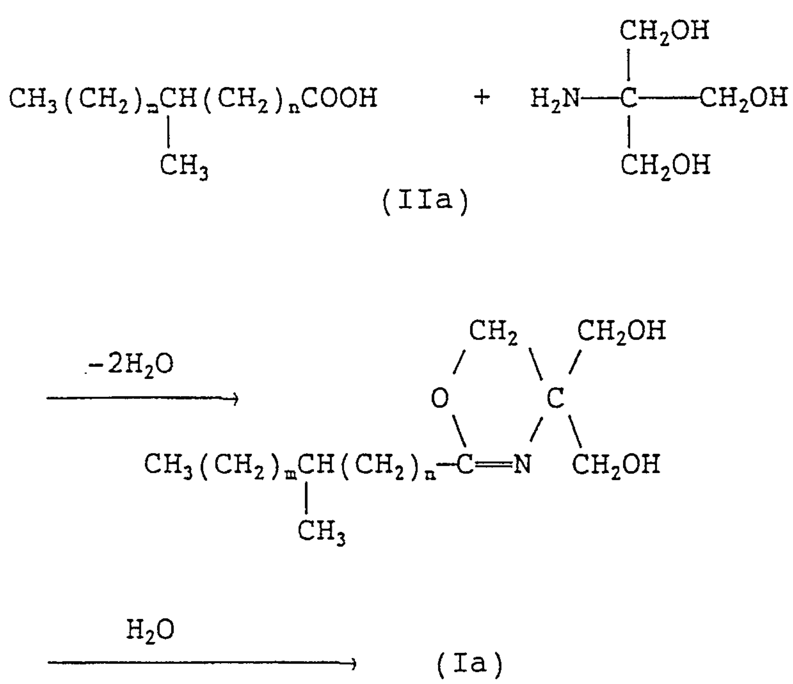


(IIa) (III) (IVa) (Ia)

In the above formulas, m and n are as defined above.

Among the branched fatty acids (II) or (11a), those which are commercially available are generally mixtures with a certain distribution with respect to the total number of carbon atoms in the alkyl moiety and with respect to the position of the branching methyl group. Methyl-branched isostearic acid, which is a byproduct in the manufacture of oleic acid dimer, contains not less than about 75% of $C_{18}$ acids (wherein m + n = 14) with $C_{14}$, $C_{16}$ and $C_{20}$ acids accounting for the balance, the branching methyl group being located approximately in the middle of the principal alkyl chain (J. Amer. Oil Chem. Soc., 51 522 (1974); ibid. 56 823A (1979). Examples thereof include, for example, Emersol® 875 (a product manufactured by

Emery) and Century® 1105 (a product manufactured by Unioncamp).

The reaction (first-step reaction) between the branched fatty acid (II) or (IIa) and tris(hydroxymethyl)-aminomethane (III) is an oxazoline ring formation reaction and these materials are preferably used in approximately equimolar amounts. This reaction is preferably carried out without using any solvent or in an inert solvent, such as toluene, xylene, mesitylene, cumene, hexane or decane, at a temperature of 100-250°C, more preferably 130-220°C, most preferably 150-200°C, while removing the byproduct water continuously. At lower temperatures, the reaction will not proceed substantially while, at higher temperatures discoloration is intense and side reactions occur. The reaction may be conducted either under a nitrogen atmosphere or under an ordinary air atmosphere. The reaction period may vary depending on various conditions. Generally, however, 1 to 24 hours is preferred.

The second-step reaction, namely hydrolysis of the oxazoline ring, is preferably carried out in a mixed solvent composed of a lower alcohol containing 1 to 6 carbon atoms and water. A method of hydrolyzing oxazolines having a straight-chain alkyl group which comprises using water alone as the solvent is disclosed in U.S. Patent No. 2,877,242. However, this method is disadvantageous in that water is required in large excess, say about 20 parts by weight per part by weight of oxazoline; smaller amounts of water lead to lower yields. The use of the above-mentioned mixed solvent according to the invention makes it possible to hydrolyze oxazolines in a small amount of solvent with a high yield.

The ratio by volume between the lower alcohol and water is preferably within the range of 1:10 to 10:1, more preferably 2:5 to 5:2, most preferably 1:2 to 2:1. When this ratio is above 10:1, the amount of water in the reaction system becomes too small and, when the ratio is below 1:10, the system becomes inhomogeneous; in both cases, the hydrolysis proceeds only slowly. The mixed solvent is preferably used in an amount of 0.1 to 20 parts by weight per part by weight of the compound (IV) or (IVa). Larger amounts decrease the productivity, hence are not practical, while smaller amounts slow down the rate of hydrolysis. As examples of the lower alcohol that are suited for use in the above hydrolysis, there may be mentioned methanol, ethanol, isopropanol, propanol, butanol, isobutanol, sec-butanol, tert-butanol, amyl alcohol, tert-amyl alcohol, neopentyl alcohol and hexyl alcohol, among others.

The hydrolysis reaction may be conducted either under a nitrogen atmosphere or under an ordinary air atmosphere. The reaction temperature should preferably be within the range of 50-150°C, more preferably 60-130°C, most preferably 70-120°C. At lower temperatures, the reaction substantially fails to progress while, at higher temperatures, discoloration becomes severe and side reactions occur. Although the reaction period may vary depending on the reaction temperature and other factors, 1 to 15 hours is generally preferred. This hydrolysis reaction may be conducted in the presence of an alkaline (e.g., hydroxides, carbonates and hydrogencarbonates of alkali metals) or acidic substance (e.g., phosphonic acid, hydrochloric acid and p-toluene sulfonic acid).

The compound (I) according to the invention can be isolated from the reaction mixture in the conventional manner, for example by solvent removal by distillation, recrystallization, chromatography and the like, employed either alone or in combination.

The branched fatty acid amides according to the invention occur as lyotropic liquid crystals in the presence of water at room temperature. Characteristically, they have excellent lubricating properties, show good compatibility with most solvents and, when admixed with water, can be dispersed almost uniformly. They are very useful as bases, emulsifiers and lubricants for hair treatment preparations and cosmetics. In accordance with the invention, the fatty acid amides can be produced in an industrially advantageous manner by using a mixed solvent composed of a lower alcohol and water in the hydrolysis step, thus allowing the reaction to proceed smoothly without requiring water in large amounts and without causing gelation.

The cosmetic composition according to the invention contains one or more of the N-tris(hydroxymethyl)-methyl-fatty acid amides mentioned above. Further, an N-tris(hydroxymethyl)methyl-fatty acid amide of formula (I) whose $R^1$ represents a linear alkenyl group containing 4 to 27 carbon atoms can also be contained in the cosmetic composition according to the invention.

That is, the N-tris(hydroxymethyl)methyl-fatty acid amide to be contained in the cosmetic composition according to the invention can be represented by the following formula (I')

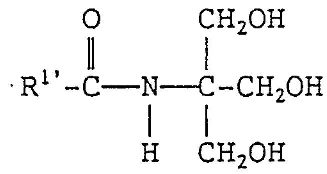

(I')

wherein, $R^{1'}$ represents a branched alkyl or linear alkenyl group each containing 4 to 27 carbon atoms.

Typical examples of the group

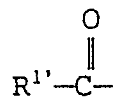

whose $R^{1'}$ is a branched alkyl group include those mentioned above as the group

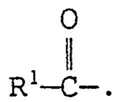

The linear alkenyl group represented by $R^{1'}$ contains preferably 10 to 22 carbon atoms and more preferably 14 to 20 carbon atoms. At typical examples of the group

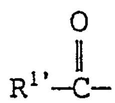

whose $R^{1'}$ is a linear alkenyl group, there may be mentioned straight-chain unsaturated acyl groups such as oleoyl group. Particularly preferred are those straight-chain alkaryl groups that are represented by formula (VII) given below:

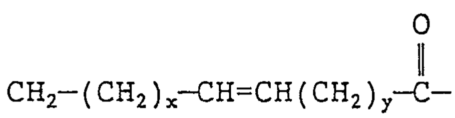

(VII)

wherein x and y each represents an integer of 0 to 24, provided that the sum of x and y is 4 to 24. Specific examples thereof include 8-heptadecenyl, 12-heneicosenyl, 9-octadecenyl and 13-docosenyl groups.

The N-tris(hydroxymethyl)methyl-fatty acid amide of formula (I') whose $R^{1'}$ is a linear alkyl group can be produced in the same manner as that of the N-tris(hydroxymethyl)methyl-fatty acid amide of formula (I) above.

The cosmetic composition according to the invention contains one or more of the N-tris(hydroxymethyl)-methyl-fatty acid amides of formula (I') above generally in amounts of 0.01 to 80% by weight preferably 0.1 to 60% by weight, based on the total weight of the composition. The amount is not critical, however.

For further heightening effects of the invention, one or more surfactants may be used additionally in the composition. Such surfactants may be nonionic, cationic, anionic or amphoteric ones. In particular, nonionic surfactants are preferred in cosmetic preparations to be applied to the skin, and cationic surfactants in hairdressing composition.

The nonionic surfactants include, among others, polyoxyethylene alkyl ethers, polyoxyethylene alkylphenyl ethers, polyoxyethylene fatty acid esters, sorbitan fatty acid esters, polyoxyethylenesorbitan fatty acid esters, fatty acid monoglycerides and glyceryl ethers.

Examples of the cationic surfactant include quaternary ammonium salts, alkylpyridinium salts, alkylmorpholinium salts, alkylisoquinolinium salts, alkylimidonium salts, alkylamine salts and alkylamideamine salts, which are commonly used in cosmetic compositions.

Of these surfactants, cationic surfactants are still preferred because the combined use of the N-tris-(hydroxymethyl)methyl-fatty acid amide with the cationic surfactant shows a favorable feel at use.

Among those cationic surfactants, quaternary ammonium salts containing at least one alkyl group having 8 to 28 carbon atoms are preferable. Examples thereof include quaternary ammonium salts of the following formulae (VIII) and (IX):

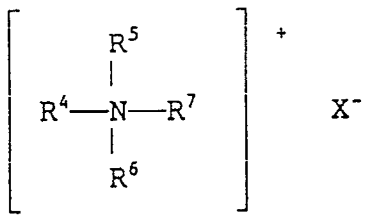

(VIII)

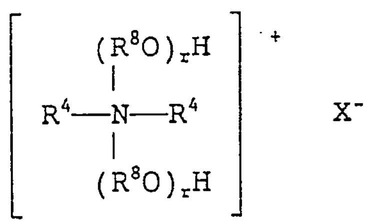

(IX)

wherein at least one of $R^4$, $R^5$, $R^6$ and $R^7$ represents an alkyl group or an alkenyl group, which may be substituted with an alkoxy group, an alkenyloxy group, an alkanoylamino group or an alkenoylamino group, where the total number of carbon atom is 8 to 28, while the others, if not such an alkyl or alkenyl group, each represent a benzyl group or an alkyl group having 1 to 5 carbon atoms or a hydroxyalkyl group having 1 to 5 carbon atoms, $R^7$ represents an alkylene group having 2 or 3 carbon atoms, $X^-$ represents a halogen ion or an organic anion and r is an integer of from 1 to 20.

The quaternary ammonium salts represented by formulae (VIII) and (IX) may be synthesized in a conventional manner as disclosed, for example, in the above mentioned Kesyohin Genryo Kijun.

Among these quaternary ammonium salts, a quaternary ammonium salt of formula (VIII) is preferable. More preferable examples of the quaternary ammonium salts of formula (VIII) include branched quaternary ammonium salts of the following general formulae (X) to (XII):

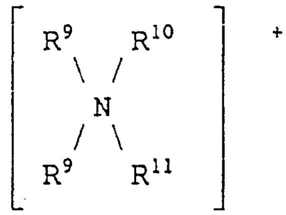

(X)

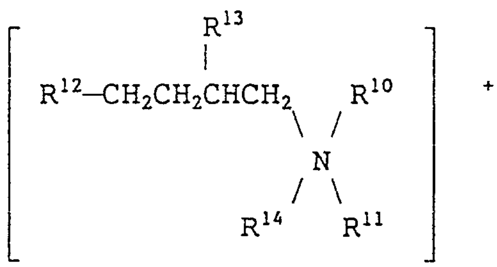

(XI)

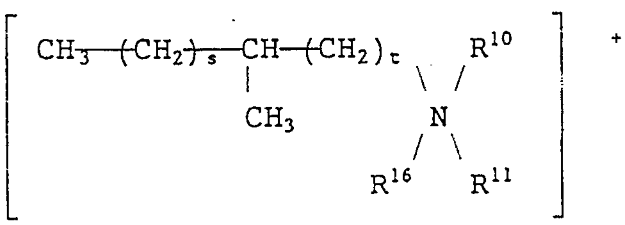

(XII)

wherein $R^9$ represents (a) a branched alkyl group of the formula:

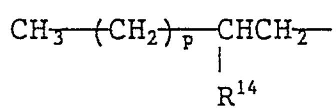

wherein $R^{15}$ represents a methyl group or an ethyl group and p is an integer which will give $R^9$ a total carbon atom number of from 8 to 16 and/or (b) a straight chain alkyl group of the formula:

$$CH_3-(CH_2)_q-$$

wherein q is an integer of from 7 to 15), $R^{10}$ and $R^{11}$ each represent a benzyl group or an alkyl group having 1 to 3 carbon atoms or a hydroxyalkyl group having 1 to 3 carbon atoms, $R^{12}$ and $R^{13}$ each represent an alkyl group having 2 to 12 carbon atoms, $R^{14}$ represents a group represented by the formula:

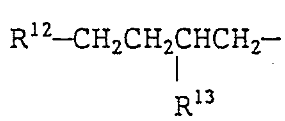

or an alkyl group having 1 to 3 carbon atoms, where $R^{12}$ and $R^{13}$ are as earlier defined, $R^{16}$ represents a group represented by the formula:

$$CH_3\!-\!(CH_2)_s\!-\!\underset{\displaystyle CH_3}{\underset{|}{CH}}\!-\!(CH_2)_t$$

or an alkyl group having 1 to 3 carbon atoms, s is an integer of from 2 to 14, t is an integer of from 3 to 11, provided that s + t is from 9 to 21, $X^-$ is a halogen ion or an organic anion, and the branching ratio ((a)/(a)+(b)) in the quaternary ammonium salt(s) represented by formula (IV) is from 10 to 100 % by mol as a whole.

The branched quaternary ammonium salt(s) represented by formula (X) may be synthesized, for example, from an oxoalcohol having 8 to 16 carbon atoms in a conventional manner as described in the above mentioned Kesyohin Genryo Kijun. Examples thereof include dialkyldimethylammonium salts, dialkylethylhydroxyethylammonium salts and dialkylmethylbenzylammonium salts each having an alkyl group derived from an oxoalcohol.

As the quaternary ammonium salt(s) of formula (X), those having the branching ratio as earlier defined of from 10 to 100 % as a whole is usually employed in the present invention. In the present invention, one quaternary ammonium salt of formula (X) may have one or two of the substituent (a) for $R^9$ and another quaternary ammonium salt of formula (X) may have one or two of the substituent (b) for $R^9$, provided that the mixture thereof as a whole suffices to the branching ratio as earlier defined.

It is particularly preferable to use those having a branching ratio as earlier defined of from 10 to 50 %. In the present invention, the total number of carbon atoms in the $R^8$ group may range from 8 to 16. It is preferable to use such quaternary ammonium salts having a specific distribution. It is more preferable to use those having the following distribution:

$C_8$ - $C_{11}$: 5 % by mol or less,
$C_{12}$: 10 - 35 % by mol,
$C_{13}$: 15 - 40 % by mol,
$C_{14}$: 20 - 45 % by mol,
$C_{15}$: 5 - 30 % by mol and
$C_{16}$: 5 % by mol or less, total being 100%.

Specific examples of such quaternary ammonium salts include dialkyldimethylammonium chloride carrying alkyl groups having 8 to 16 carbon atoms and having a branching ratio as earlier defined of from 10 to 50 %.

The branched quaternary ammonium salts represented by formula (XI) may be synthesized in a conventional manner from a Guerbet alcohol

$$(R^{12}\!-\!CH_2CH_2\overset{\displaystyle R^{13}}{\overset{|}{C}}HCH_2OH,$$

where $R^{12}$ and $R^{13}$ are as earlier identified) having 8 to 28 carbon atoms. Preferable examples of the branched quaternary ammonium salt include alkyltrimethylammonium salts, alkyldimethylbenzylammonium salts, dialkyldimethylammonium salts, dialkylmethylhydroxyethyl ammonium salts and dialkylmethylbenzylammonium salts, each having an alkyl group derived from a Guerbet alcohol. Among these substances, 2-decyltetradecyltrimethylammonium chloride, 2-dodecylhexadecyltrimethylammonium chloride, di-2-hexyldecyldimethylammonium chloride and di-2-octyldodecyldimethylammonium chloride are particularly preferable.

As the methyl branched quaternary ammonium salt represented by formula (XII), those wherein s + t is 15 are preferable.

The methyl branched quaternary ammonium salt represented by formula (XII) may be synthesized by the manner described, for example, in The fifteenth IFSCC International Congress (1988), Preprints, vol.1, pages 71 to 87.

Examples of the counter ion $X^-$ in the quaternary ammonium salts represented by formulae (XIII), (IX), (X), (XI) and (XII) include halogen ions (for example, chlorine, iodine, bromine ions); and organic anions (for example, methosulfate, ethosulfate, methophosphate, ethophosphate).

These surfactants are used in amounts of 0.01 to 30% by weight, preferably 0.1 to 10% by weight, on the basis of the whole composition.

The cosmetic composition according to the invention may further contain, if necessary or where appropriate, one or more of those ingredients that are generally used in cosmetics, drugs and foods, for example higher alcohols having a straight or branched alkyl or alkenyl group; hydrocarbons such as liquid paraffin, vaseline and solid paraffin; lanolin and derivatives such as liquid lanolin and lanolin fatty acids; silicone derivatives such as dimethylpolysiloxane, polyether-modified polysiloxanes, amino-modified polysiloxanes and stearoxy-modified siloxanes; fats and oils such as higher alcohol higher fatty acid esters, higher fatty acids and alkyl- or alkenyl-containing long-chain amideamines; and animal- or plant-derived fats and oils such as mink oil and olive oil.

In particular, the silicone derivatives can preferably be contained in the cosmetic composition of the invention since the combined use of the N-tris(hydroxymethyl)methyl-fatty acid amide with the silicone derivative shows a favorable feel during use.

Examples of the silicone derivative are as follows:

(1) dimethyl polysiloxane:

$(CH_3)_3SiO[(CH_3)_2SiO]_{n'}Si(CH_3)_3$ (XIII)

wherein n' is from 0 to 9,000,

(2) methylphenyl polysiloxane:

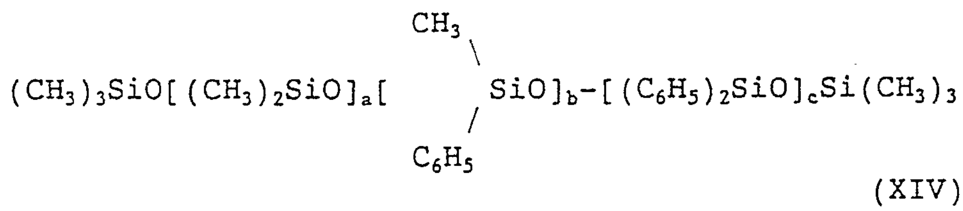

(XIV)

wherein a+b+c is 1 to 9,000 provided that when b is 0, c is not 0 and when c is 0, b is not 0,

(3) amino-denatured silicone:

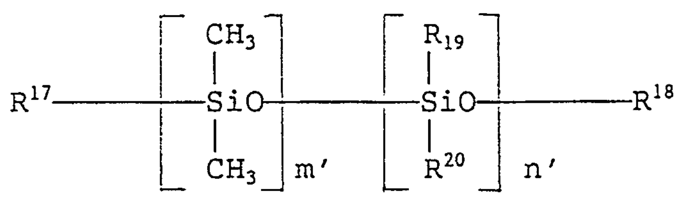

(XV)

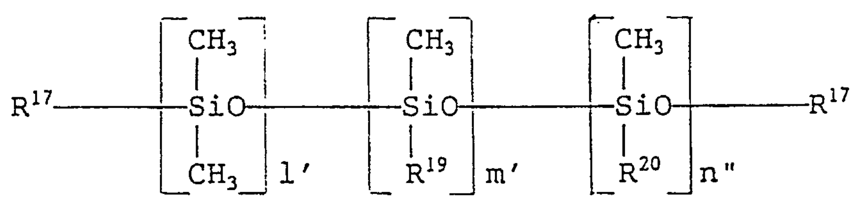

(XVI)

wherein $R^{17}$ is a methyl or hydroxy group, and $R^{18}$ is a methyl group or a hydrogen, $R^{19}$ is the following aminoalkyl group (XVII):

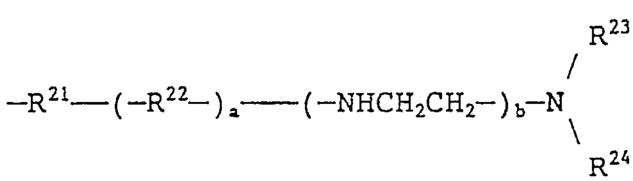

(XVII)

wherein $R^{21}$ represents a divalent hydrocarbon group, $R^{22}$ represents a group $-OCH_2CH_2-$,

$$-O\underset{\underset{\displaystyle CH_3}{|}}{C}HCH_2-,$$

$$-OCH_2\underset{\underset{\displaystyle OH}{|}}{C}HCH_2-,\ \text{or}\ -OCH_2\underset{\underset{\displaystyle CH_2OH}{|}}{C}H-,$$

$R^{23}$ and $R^{24}$ are individually a hydrogen or a monovalent hydrocarbon group, and a and b denote integers of 1-6, $R^{20}$ is an alkyl group, a hydroxy, hydroxyalkyl, oxyalkylene or polyoxyalkylene group, and l', m' and n'' are integers dependent on the molecular weight.

(4) fatty acid-denatured polysiloxane:

$$(CH_3)_3SiO\left[\begin{array}{c}CH_3\\|\\SiO\\|\\CH_3\end{array}\right]_f\left[\begin{array}{c}CH_3\\|\\SiO\\|\\(CH_2)_iOCOR^{25}\end{array}\right]_g\left[\begin{array}{c}(CH_2)_iOCOR^{25}\\|\\SiO\\|\\(CH_2)_iOCOR^{25}\end{array}\right]_h Si(CH_3)_3 \qquad (XVIII)$$

wherein f, g and h each represents 1 to 350, i is 0 to 10, and $R^{25}$ represents a group

$$C_{n_1}H_{2n_1+1},$$

in which $n_1$ is 9 to 21,

(5) alcohol-denatured silicone:

$HO(CH_2)\cdot R^{26}-[(CH_3)_2SiO]_j(CH_3)_2SiR^{26}-CH_2OH$ (XIX-1)

$$(CH_3)_3SiO[(CH_3)_2SiO]_j(CH_3\underset{\underset{\displaystyle R^{26}-\underset{\underset{\displaystyle CH_3}{|}}{CH}\cdot OH}{|}}{Si}O)_kSi(CH_3)_3 \qquad (XIX-2)$$

wherein j and k each represents 1 to 500, preferably 1 to 200; and $R^{26}$ represents a group

$$C_{n_2}H_{2n_2},$$

in which $n_2$ is 0 to 4,

(6) aliphatic alcohol-denatured polysiloxane:

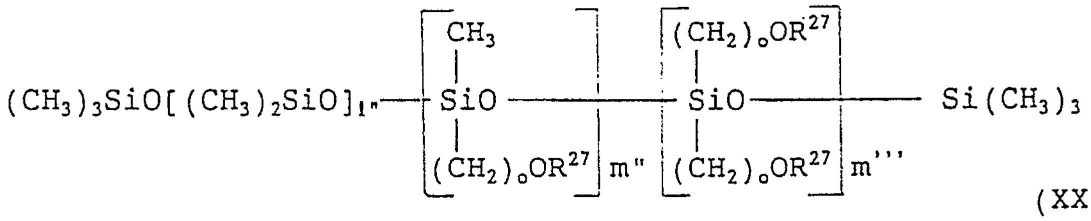

(XX)

wherein ℓ"+m"+n is 1 to 300, o is 0 to 5, and $R^{27}$ represents a group

$C_{n_3}H_{n_3+1}$,

in which $n_3$ is 4 to 22.

(7) polyether-denatured silicone:

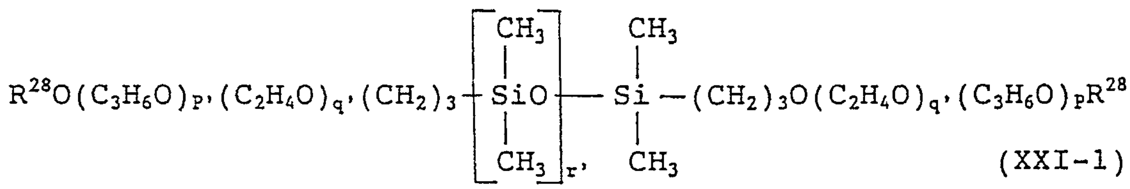

(XXI-1)

wherein p' is 0 to 35, q' is 1 to 45, r' is 0 to 400, and $R^{28}$ represents a group $C_{n'''}H_{2n'''+1}$, in which n''' is 1 to 4,

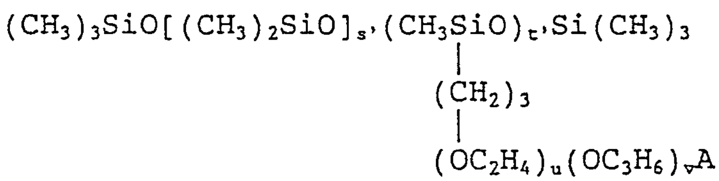

(XXI-2)

wherein s' is 1 to 100, preferably 20 to 80; t' is 1 to 50, preferably 3 to 30; u is 0 to 50, preferably 5 to 30; v is 0 to 50, preferably 0 to 35; and A is an alkyl group having 1 to 12 carbon

atoms

or a group $OC_nH_{2n+1}$, in which $n_4$ is 0 to 6,

(8) epoxy-denatured silicone:

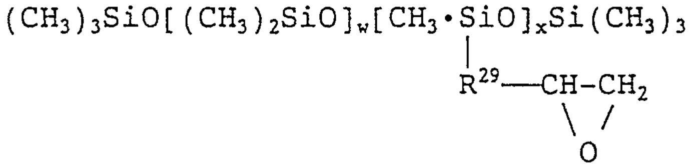

(XXII)

wherein w is 1 to 500, preferably 1 to 250; x is 1 to 50, preferably 1 to 30; and $R^{29}$ represents an alkylene group having 1 to 3 carbon atoms,

(9) fluorine-denature silicone:

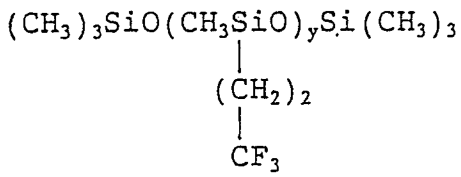

(XXIII)

wherein y is 1 to 400, preferably 1 to 250,
(10) cyclic silicone:

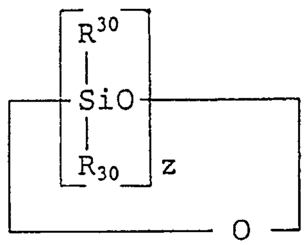

(XXIV)

wherein z is 3 to 8, and $R^{30}$ represents an alkyl group having 1 to 3 carbon atoms, and
(11) alkyl-denatured silicone:

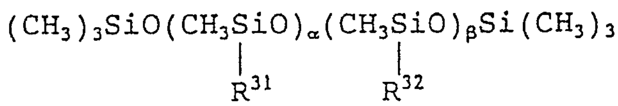

(XXV-1)

wherein $\alpha$ and $\beta$ each represents 1 to 500, preferably 1 to 200; $R^{31}$ represents an alkyl group having 2 to 18 carbon atoms; and $R^{32}$ is a group

$$C_{n_5}H_{2n_5}$$

in which $n_5$ is 0 to 4,

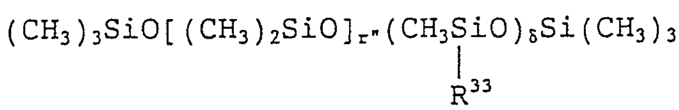

(XXV-2)

wherein r'' and $\delta$ each represents 1 to 500, preferably 1 to 200; and $R^{33}$ represents an alkyl group having 10 to 16 carbon atoms,
(12) alkoxy-denatured silicone:

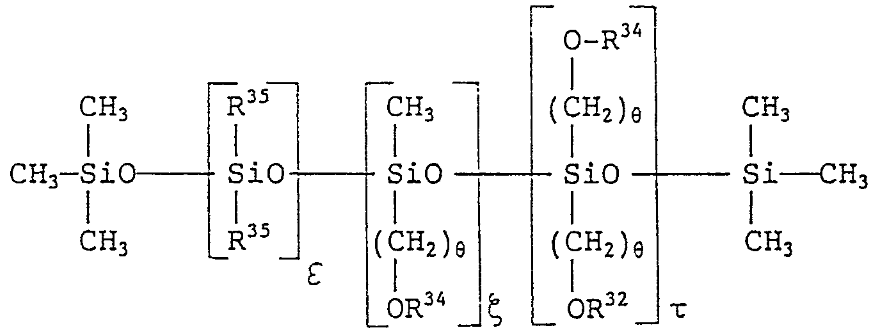

wherein $R^{34}$ represents an alkyl group having 1 to 28 carbon atoms; $R^{35}$ represents methyl group or phenyl group; $\epsilon$ is 1 to 3,000; $\zeta + \tau$ is 1 to 500; and $\theta$ is 0 to 6.

Among these silicone compounds, those as defined in the above (1), (3), (7) and (10) are preferably employed in a hairdressing composition to be rinsed away, for example, a hair rinse or a hair conditioner. Regarding the silicone compounds of (1), the n' in the formula (XIII) may range from 0 to 9,000, though it preferably ranges from 100 to 1,000 in order to give a hair style of a light feel. On the other hand, those as defined in the above (1), (2), (3), (7) and (10) are preferably employed in a hairdressing composition which is not rinsed away, such as a hair cream, a styling lotion or a styling mousse. Regarding the silicone compounds of (1), the n' in the formula (XIII) preferably range from 6,000 to 8,000 in order to minimize any oily feel.

The cosmetic composition according to the invention may still further contain, if necessary or where appropriate, one or more of those ingredients that are generally used in cosmetics, drugs and food, for example, biologically active agents such as antidandruff agents, biocides and vitamins; preservatives such as parabens; thickening agents such as water-soluble macromolecules, colorants such as dyes and pigments; ultraviolet absorbers; astringents; humectants such as propylene glycol, glycerin, carbitols, 3-methyl-1,3-butanediol and sugars; water and perfumes.

The cosmetic composition according to the invention can be manufactured in a conventional manner and can be provided for example in the form of skin care preparations of the oil-in-water or water-in-oil emulsion type or of the oleaginous type; make-up preparations such as rouge and foundations; and hair cosmetics such as hair rinses, hair treatment preparations, hair conditioners and hair sprays.

Since the N-tris(hydroxymethyl)methyl-fatty acid amides serve as excellent humectants and occur as lamellar liquid crystals in the presence of water, the cosmetic composition according to the invention has good spreadability or extendibility and gives only a minimum of greasy feeling. Furthermore, since the liquid crystals take in high-melting substances, the stability of the system is improved and the active ingredients can function efficiently. Therefore, the cosmetic composition according to the invention is excellent in humectancy and, when applied, can spread smoothly, giving a refreshing feeling during use without stickiness. Furthermore, the composition has good emulsion stability.

The following examples are further illustrative of the present invention but by no means limitative of the scope thereof.

EXAMPLE 1

Synthesis of N-tris(hydroxymethyl)methylisostearamide (Compound (Ia-1)):

(1) iso-Heptadecyl-4,4-bis(hydroxymethyl)-2-oxazoline (Compound (IVa-1)):

A one-liter reactor equipped with a thermometer, a reflux condenser, a Dean-Stark trap and a stirrer was charged with 300 g (1.055 moles) of isostearic acid (Emersol® 875; trade name of a product manufactured by Emery) and 127.75 g (1.055 moles) of tris(hydroxymethyl)aminomethane. The temperature was raised with stirring and the reaction was carried out at 140-160 °C for 3 hours and then further at 160-196 °C for 3 hours, whereby the calculated amount of water was distilled off to give 386.25 g of the title compound (IVa-1) (yield 99.1%). This product occurred as a viscous yellow liquid at 140 °C and solidified into a wax at room temperature. Melting point 53-58 °C.

Purity determined by gas chromatography: 90.7%

(2) N-Tris(hydroxymethyl)methylisostearamide (Compound (Ia-1)):

A one-liter reactor equipped with a thermometer, a reflux condenser and a stirrer was charged with 150 g (0.368 mole) of compound (IVa-1) obtained as described above and 300 ml of ethanol. The

temperature was raised with stirring and, at 76° C, 450 ml of water was added dropwise to the reactor over 15 minutes. The reaction mixture became cloudy. After 5 hours of refluxing at 83-84° C with stirring, the reaction mixture was cooled to room temperature, whereupon the reaction mixture became almost clear and transparent. The mixture was dehydrated by several repetitions of evaporation following addition of ethanol to give 157.67 g of a crude form of the compound (Ia-1). This crude product had a purity of 87.6% as determined by gas chromatography. The product was dissolved in 1,000 ml of chloroform, the insoluble matter was removed, the filtrate was evaporated, and the residue was recrystallized from 500 ml of hexane. The solid thus obtained was washed with hexane and dried under vacuum at 50° C. Thus was obtained 79.62 g of the compound (Ia-1) (yield 55.8%).

Purity determined by gas chromatography: 95.2%

At room temperature, this product occurred as a colorless paste solid.

Acid value: 1.42

Hydroxyl value: 414.1 (theoretical value: 434.26)

IR (KBr, $cm^{-1}$) (cf. Fig. 1):

3380 (O-H stretching), 3300 (N-H stretching), 2928, 2860 (C-H stretching), 1626 (C=O stretching), 1536 (N-H deformation), 1468, 1380 (C-H deformation), 1050, 1026 (C-O stretching), 722 ($CH_2$ rocking), 668 (N-H out-of-plane deformation)

NMR ($CDCl_3$, δ ppm) (cf. Fig. 2):

0.65 - 1.05 (6H, broad, $-CH_3$), 1.05 - 2.02 (27H, broad, $-CH_2-$,

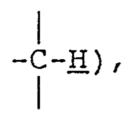

2.02 - 2.58 (2H, broad,

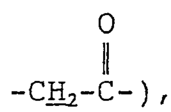

3.32 - 3.99 (6H, broad, $-CH_2OH$), 4.77 - 5.48 (3H, broad, $-CH_2OH$, 6.35 - 6.65 (1H, broad,

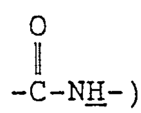

EXAMPLE 2

N-Tris(hydroxymethyl)methylisostearamide (Ia-1) obtained in Example 1 and several known compounds similar in structure, namely two N-tris(hydroxymethyl)methyl-straight chain fatty acid amides and four trimethylol-alkanes, were examined for appearance at room temperature and for compatibility with water. The test compound-water mixture systems gave the results shown in Table 1 over the entire concentration range.

TABLE 1

| | Test compound | Appearance (room temperature) | Compatibility with water |
|---|---|---|---|
| Compound according to the invention | N-Tris(hydroxymethyl)-methylisostearamide (Ia-1) | Paste solid | Homogeneous dispersion |
| Comparison Compound | N-Tris(hydroxylethyl)-methyllauramide | Solid | Solid-liquid separation |
| Comparison Compound | N-Tris(hydroxymethyl)-methylstearamide | Solid | Solid-liquid separation |
| Comparison Compound | Trimethylolpropane | Solid | Homogeneous dispersion |
| Comparison Compound | Trimethylolnonane | Solid | Solid-liquid separation |
| Comparison Compound | Trimethylolheptadecane | Solid | Solid-liquid separation |
| Comparison Compound | Trimethylolisoheptane | Viscous liquid | Solid-liquid separation |

EXAMPLE 3

An 80% by weight aqueous solution of each humectant sample was prepared, about 1.5 g of the solution was placed in a dish having a diameter of 30 mm and allowed to stand in a $P_2O_5$ desiccator at

room temperature and the decrease in the weight of the solution was measured by a balance followed at timed intervals for evaluating the water-retaining capacity.

(Samples)

| | |
|---|---|
| Composition 1 according to the invention: | N-Tris(hydroxymethyl)methylisostearamide (obtained in Example 1) |
| Composition for comparison 1: | Propylene glycol |
| Composition for comparison 2: | Glycerin |

The results are shown in Fig. 3. The composition according to the invention showed a superior water-retaining capacity as compared with the conventional humectants.

EXAMPLE 4

Using the compound according to the invention as produced in Example 1, a hair rinse composition (composition 2 according to the invention) was prepared according to the formulation given in Table 2 and evaluated for its rinsing effect.

(Production Procedure)

The ingredients shown in Table 2 were melted by heating at 70°C and added to water heated at the same temperature. The resulting mixture was stirred to thoroughly mix the ingredients and then cooled to room temperature with stirring to give a hair rinse composition. (Evaluation)

Twenty grams (20 g) of hair (15 cm long) from a Japanese female who had not undergone cosmetic treatment such as cold waving, bleaching or the like were bundled, shampooed with an anionic surfactant-based commercial shampoo and evenly coated with 2 g of the test hair rinse composition specified in Table 2. The coated hair was rinsed in running water for 30 seconds and then towel-dried. The hair bundle in a wet condition was subjected to sensory evaluation for softness, smoothness and greasy feeling according to the following criteria: very good-A, good-B, average-C, poor-D. The results are shown in Table 2.

TABLE 2

| Composition | The invention (% by weight) | Control 1 (% by weight) | Control 2 (% by weight) | Control 3 (% by weight) |
|---|---|---|---|---|
| N-Tris(hydroxymethyl) methylisostearamide Compound (Ia-1) according to the invention) | 3.0 | - | - | - |
| Isostearyl alcohol | - | 3.0 | - | - |
| Isostearyl monoglyceride | - | - | 3.0 | - |
| Stearyltrimethyl-ammonium chloride | 2.0 | 2.0 | 2.0 | 2.0 |
| Water | 95.0 | 95.0 | 95.0 | 95.0 |
| Sensory evaluation Effects on hair | | | | |
| (1) Softness | B | D | C | B |
| (2) Smoothness | A | D | D | C |
| (3) Less in greasy feeling | A | C | B | B |

The hair rinse composition containing N-tris(hydroxymethyl)methylisostearamide according to the invention was superior in softness- and smoothness-imparting properties and gave a minimum of greasy feeling.

EXAMPLE 5

Hair treatment composition (composition 3 according to the invention):

| | | (% by weight) |
|---|---|---|
| 1) | Stearyltrimethylammonium chloride | 2.0 |

| | | |
|---|---|---|
| 2) | Dimethylpolysiloxane (500 cs) | 1.0 |
| 3) | Cetostearyl alcohol | 3.0 |
| 4) | N-Tris(hydroxymethyl)methyliso-stearamide (obtained in Example 1) | 3.0 |
| 5) | Liquid paraffin | 3.0 |
| 6) | Hydroxyethylcellulose (viscosity of 1% aqueous solution: 8,000 cp) | 0.5 |
| 7) | Polyoxyethylene oleyl ether (5 E.O.) | 0.5 |
| 8) | Methylparaben | 0.2 |
| 9) | Perfume | 0.4 |
| 10) | Water | Balance |
| | Total | 100.0 |

The above formulation gave a hair treatment composition excellent in smoothing and softening properties and capable of providing hair with a light, moist, good feeling without giving any significant greasy feeling.

EXAMPLE 6

Hair cream composition (composition 4 according to the invention):

| | | (% by weight) |
|---|---|---|
| 1) | Di-2-hexyldecyldimethylammonium chloride | 2.0 |
| 2) | Cetyltrimethylammonium chloride | 1.0 |
| 3) | N-Tris(hydroxymethyl)methyliso-stearamide (obtained in Example 1) | 1.0 |
| 4) | Cetyl alcohol | 5.0 |
| 5) | Dipropylene glycol | 6.0 |

| | | |
|---|---|---|
| 6) | Glycerin | 10.0 |
| 7) | Liquid paraffin | 3.0 |
| 8) | Perfume | 0.4 |
| 9) | Water | Balance |
| | Total | 100.0 |

The above formulation gave a hair cream composition excellent in smoothing and softening properties and capable of providing hair with a good feeling without stickiness.

EXAMPLE 7

Styling lotion composition (composition 5 according to the invention):

| | | (% by weight) |
|---|---|---|
| 1) | 2-Dodecylhexadecyltrimethylammonium chloride | 0.5 |
| 2) | N-Tris(hydroxymethyl)methylisostearamide (obtained Example 1) | 0.2 |
| 3) | Methacrylate polymer (Note 1) | 1.0 |
| 4) | Polyethylene glycol 6000 | 1.0 |
| 5) | Ethanol | 20.0 |
| 6) | Perfume | 0.3 |
| 7) | Water | Balance |
| | Total | 100.0 |

Note 1: YUKAFORMER AM-75 (trade name of a product manufactured by Mitsubishi Petrochemical Co., Ltd.)

The above formulation gave a setting composition having good feel and capable of providing hair with the ability to retain a hair style satisfactorily.

EXAMPLE 8

Conditioning mousse composition (composition 6 according to the invention):

| | | (% by weight) |
|---|---|---|
| 1) | Dialkyldimethylammonium chloride (Note 2) | 0.5 |
| 2) | Methylphenylpolysiloxane (300 cs) | 1.0 |
| 3) | Isotridecyl myristate | 1.0 |
| 4) | 3-Methyl-1,3-butanediol | 1.0 |
| 5) | Glycerin | 2.5 |
| 6) | Liquid paraffin | 2.5 |
| 7) | N-Tris(hydroxymethyl)methyliso-stearamide (obtained in Example 1) | 0.2 |
| 8) | 95% Ethyl alcohol | 5.0 |
| 9) | methylparaben | 0.1 |
| 10) | Perfume | 0.1 |
| 11) | Propellant (LPG) | 10.0 |
| 12) | Water | Balance |
| | Total | 100.0 |

Note 2: A branched quaternary ammonium salt mixture (degree of branching = 20%) derived from a mixture of commercial $C_{12-15}$ alcohols synthesized by the oxo process (1:1 mixture of DOBANOL 23 and DOBANOL 45, manufactured by Mitsubishi Petrochemical Co., Ltd.).

The above formulation gave a conditioning mousse composition capable of providing hair with a good feel.

EXAMPLE 9

Cream (composition 7 according to the invention)

Oil phase components:

| | (% by weight) |
|---|---|
| Cetanol | 2.0 |
| Stearic acid | 3.0 |
| N-Tris(hydroxymethyl)methyliso-stearamide (obtained in Example 1) | 3.0 |
| Cholesteryl isostearate | 8.0 |
| Monolaurylglycerin | 2.0 |
| Polyoxyethylenesorbitan monolaurate (20 E.O.) | 2.0 |

Water phase components:

| | (% by weight) |
|---|---|
| Dipropylene glycol | 10.0 |
| 1,3-Butylene glycol | 5.0 |
| Ethylparaben | 0.1 |
| Methylparaben | 0.2 |
| Perfume | 0.1 |
| Water | Balance |
| Total | 100.0 |

The cream according to the invention had an excellent feel during use and showed a good humectant effect.

EXAMPLE 10

Milky lotion (composition 8 according to the invention)

Oil phase components:

| | (% by weight) |
|---|---|
| Cetanol | 0.5 |
| Vaseline | 1.0 |
| N-Tris(hydroxymethyl)methyliso-stearamide (obtained in Example 1) | 10.0 |
| Polyoxyethylene monooleate (10 E.O.) | 2.0 |
| Stearic acid | 2.0 |

Water phase components:

| | (% by weight) |
|---|---|
| 1,3-Butylene glycol | 3.0 |
| Dipropylene glycol | 6.0 |
| Triethanolamine | 1.0 |
| Ethylparaben | 0.1 |
| Methylparaben | 0.2 |
| Perfume | 0.1 |
| Water | Balance |
| Total | 100.0 |

The milky lotion according to the invention showed a good and long-lasting humectant effect.

EXAMPLE 11

Cosmetic base (composition 9 according to the invention)

| | (% by weight) |
|---|---|
| Glycerin | 15 |
| Polyoxyethylene octyl dodecyl ether (20 E.O.) | 10 |
| N-Tris(hydroxymethyl)methyliso stearamide (obtained in Example 1) | 10 |
| Squalane | 50 |
| Water | Balance |
| Total | 100 |

The above ingredients were made up into a one-phase cosmetic base by heating all the ingredients for dissolution, followed by mixing and cooling. The cosmetic base had an excellent feel during use and showed a good humectant effect. Various oil-soluble substances can be incorporated into the base.

EXAMPLE 12

Emulsion-type foundation (composition 10 according to the invention):

Oil phase components:

| | (% by weight) |
|---|---|
| Stearic acid | 5.0 |
| Cetostearyl alcohol | 1.0 |
| N-Tris(hydroxymethyl)methylheptyl-undecanamide | 0.5 |

| | |
|---|---|
| N-Tris(hydroxymethyl)methyliso-stearamide (obtained in Example 1) | 6.0 |
| Cholesteryl isostearate | 6.0 |
| Glycerin monolaurate | 2.0 |
| Propylene glycol monolaurate | 3.0 |

Water phase components:

| | (% by weight) |
|---|---|
| Glycerin | 10.0 |
| Triethanolamine | 1.2 |
| Ethylparaben | 0.1 |
| Methylparaben | 0.1 |
| Perfume | 0.1 |
| Water | Balance |

Powder components:

| | (% by weight) |
|---|---|
| Titanium oxide | 8.0 |
| Talc | 4.0 |
| Iron oxide | 0.5 |
| Total | 100.0 |

The foundation according to the invention has an excellent feel during use and showed a good humectant effect.

EXAMPLE 13

Rouge or lipstick (composition 11 according to the invention):

| | | (% by weight) |
|---|---|---|
| 1) | Microcrystalline wax | 6.0 |
| 2) | Candelilla wax | 3.0 |
| 3) | Glycerin diisostearate | 5.0 |
| 4) | N-Tris(hydroxymethyl)methyliso-stearamide (obtained in Example 1) | 10.0 |
| 5) | Cerebroside (manufactured by Cedary Research Laboratories) | 2.0 |
| 6) | Jojoba oil | 6.0 |
| 7) | Olive oil | Balance |
| 8) | Lanolin | 10.0 |
| 9) | Pigment | 7.0 |
| 10) | Perfume | 0.1 |
| | Total | 100.0 |

The rouge according to the invention had a good feel during use and showed an excellent humectant effect.

EXAMPLE 14

Cleansing cream (composition 12 according to the invention):

| | (% by weight) |
|---|---|
| Sorbitol | 10 |
| Polyoxyethylene octyl dodecyl ether (25 E.O.) | 15 |

| | |
|---|---|
| N-Tris(hydroxymethyl)methyliso-stearamide (obtained in Example 1) | 60 |
| Glycerin | 5 |
| Dibutylhydroxytoluene | 0.1 |
| Perfume | 0.1 |
| Water | Balance |
| Total | 100.0 |

The cleansing cream according to the invention was very stable and showed an excellent cleansing effect.

EXAMPLE 15

Synthesis of N-tris(hydroxymethyl)methyl-α-methylstearamide (Compound (Ia-2)) represented by the formula

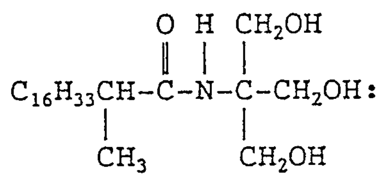

(1) 2-(1'-Methylheptadecyl)-4,4-bis(hydroxymethyl)-2-oxazoline (Compound (IVa-2)):

A 300 ml reactor equipped with a thermometer, a reflux condenser, a Dean-Stark trap and a stirrer was charged with 103.2 g (0.346 mole) of α-methylstearic acid and 42.4 g (0.350 mole) of tris-(hydroxymethyl)aminomethane. The temperature was raised with stirring and the reaction was carried out at 160 °C for 15 hours and then further at 140-160 °C for 51 hours, whereby the calculated amount of water was distilled off to give 131.40 g of the title compound (IVa-2) (yield 99.0%). This product occurred as a viscous yellow liquid at 140 °C and solidified into a wax at room temperature. Melting point 44-45 °C.

IR (KBr, $cm^{-1}$):

3404 (O-H stretching), 2924, 2856 (C-H stretching), 1664 (C-H stretching), 1470, 1382 (C-H deformation), 1142 (C-O-C stretching), 1028 (C-O stretching), 724 ($CH_2$ rocking)

NMR ($CDCl_3$, δ ppm): 0.88 (6H, triplet, J=6Hz, $-\underline{CH_3}$), 0.95 - 1.80 (30H, broad, $-CH_2-$), 2.30-2.60 (1H, broad,

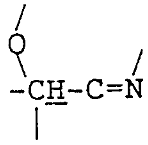

),

2.90 - 3.90 (2H, broad, $-CH_2OH$),3.62 (4H, doublet × doublet, J=12.4Hz, 14.6Hz, $-\underline{CH_2}OH$), 4.14 (2H, singlet,

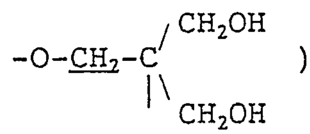

(2) N-Tris(hydroxymethyl)methyl-α-stearamide (Compound (Ia-2)):

A 200 ml reactor equipped with a thermometer, a reflux condenser and a stirrer was charged with 105.2 g (0.274 mole) of compound (IVa-2) obtained as described above and 15 ml of ethanol. The temperature was raised with stirring and, at 90 ° C, 14.7 g (0.817 mole) of water was added dropwise to the reactor over 40 minutes. The reaction mixture became cloudy. After 21 hours of refluxing at 90-110 ° C with stirring, the reaction mixture was cooled to room temperature. The mixture was dehydrated by several repetitions of evaporation following addition of ethanol to give a crude form of the compound (Ia-2). The crude product thus obtained was dissolved in 500 ml of chloroform while heating, the insoluble matter was removed, and then recrystallization was conducted. The solid thus obtained was washed with chloroform and dried under vacuum at 40 ° C. Thus was obtained 39.1 g of the compound (Ia-2) (yield 35.5%). Melting point 108 - 111 ° C.

Purity determined by gas chromatography: 93.6%

Acid value: 5.1

Hydroxyl value: 406.2 (theoretical value: 434.26)

IR (KBr, $cm^{-1}$):

3324 (O-H stretching, N-H stretching), 2920, 2852 (C-H stretching), 1628 (C=O stretching), 1528 (N-H deformation), 1468, 1378 (C-H deformation), 1048, 1024 (C-O stretching), 720 ($CH_2$ rocking), 670 (N-H out-of-plane deformation)

NMR ($CDCl_3$, δ ppm):

0.88 (3H, triplet, J=6.6Hz, $-CH_3$), 1.00 - 1.80 (30H, broad, $-CH_2-$), 1.13 (3H, doublet, J=6.8Hz,

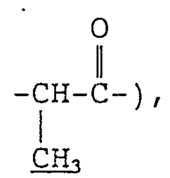

2.23 (1H, sextet, J=6.8Hz,

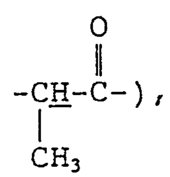

3.56 (9H, singlet, $-CH_2OH$, $-CH_2OH$), 6.52 (1H, singlet,

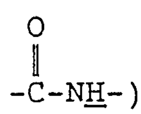

EXAMPLE 16

Synthesis of N-tris(hydroxymethyl)methyl-α-octylarachamide (Compound (Ia-3)) represented by the formula

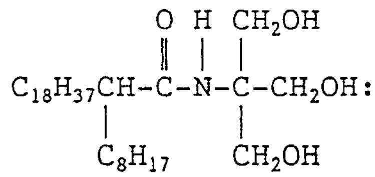

(1) 2-(1'-Octyleicosy)-4,4-bis(hydroxymethyl)-2-oxazoline (Compound (IVa-3)):

A 300 ml reactor equipped with a thermometer, a reflux condenser, a Dean-Stark trap and a stirrer was charged with 100 g (0.235 mole) of α-octylarachic acid and 27.74 g (0.229 mole) of tris-(hydroxymethyl)aminomethane. The temperature was raised with stirring and the reaction was carried out at 160-179 °C for 55 hours, whereby the calculated amount of water was distilled off to give 112.15 g of the title compound (IVa-3) (yield 96.1%).

Purity determined by gas chromatography: 93.2%

IR (NEAT, $cm^{-1}$):

3292 (O-H stretching), 2928, 2860 (C-H stretching), 1660 (C=N stretching), 1466, 1380 (C-H deformation), 1125 (C-O-C stretching), 1054, 1000 (C-O stretching), 722 ($CH_2$ rocking)

NMR ($CDCl_3$, δ ppm):

0.88 (6H, triplet, J=6.2Hz, -$CH_3$), 1.00 - 1.80 (48H, broad, -$CH_2$-); 1.80 - 2.20 (2H, broad, -$CH_2OH$), 2.20 - 2.60 (1H, broad,

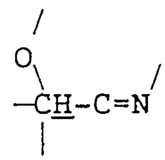

),

3.64 (4H, doublet × doublet, J=11.6Hz, 18.8Hz, -$CH_2OH$), 4.16 (2H, singlet,

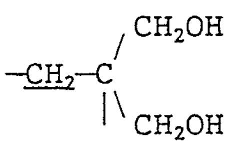

)

(2) N-Tris(hydroxymethyl)methyl-α-octylarachamide (Compound (Ia-3)):

A 300 ml reactor equipped with a thermometer, a reflux condenser and a stirrer was charged with 80 g (0.157 mole) of compound (IVa-3) obtained as described above and 8.5 ml of ethanol. The temperature was raised with stirring and, at 90 °C, 8.47 g (0.471 mole) of water was added dropwise to the reactor over 5 minutes. The reaction mixture became cloudy. After 92 hours of refluxing at 83-117 °C with stirring, the reaction mixture was cooled to room temperature. The mixture was dehydrated by several repetitions of evaporation following addition of ethanol to give 80.66 g of a crude form of the compound (Ia-3). The product was purified by means of a silica gel column chromatography using chloroform/methanol. Thus was obtained 24.86 g of the compound (Ia-3) (yield 30.3%). Melting point 57-63 °C.

Purity determined by gas chromatography: 95.2%

Acid value: 2.03

Hydroxyl value: 288.2 (theoretical value: 318.87)

IR (NEAT, $cm^{-1}$):

3344 (O-H stretching, N-H stretching), 2924, 2856 (C-H stretching), 1628 (C=O stretching), 1526 (N-H deformation), 1464, 1380 (C-H deformation), 1056, 1028 (C-O stretching), 720 ($CH_2$ rocking)

NMR ($CDCl_3$, δ ppm):

0.88 (6H, triplet, J=6.7Hz, -$CH_3$), 1.00 - 1.80 (48H, broad, -$CH_2$), 1.90 - 2.20 (1H, broad,

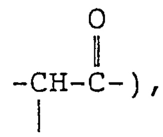

3.58 (9H, singlet, -C<u>H</u>$_2$OH, -CH$_2$O<u>H</u>), 6.49 (1H, singlet,

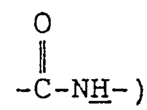

EXAMPLE 17

Synthesis of N-tris(hydroxymethyl)methyl-α-heptylundecanamide (Compound (Ia-4)) represented by the formula:

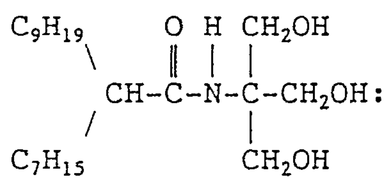

A 200 ml reactor equipped with a thermometer, a reflux condenser and a stirrer was charged with 50 g (0.176 mole) of α-heptylundecanic acid (Diadol; trade name of a product manufactured by Mitsubishi Kasei Corporation) and 63.96 g (0.528 mole) of tris(hydroxymethyl)aminomethane. The temperature was raised with stirring and the reaction was carried out at 150-170 °C for 34 hours and then the reaction mixture was cooled to 100 °C followed by the addition of 100 g of water. The reaction mixture became cloudy. After 24 hours of refluxing at 98 °C, the reaction mixture was cooled to room temperature. After adding 50 ml of water, the mixture was extracted thrice with ether (200 ml, 100 ml and 100 ml). These ether layers were washed three times with a saturated aqueous solution of $NaHCO_3$ and two times with a saturated aqueous solution of common salt, and then dried over Glauber's salt followed by evaporation to thereby give 56.29 g of a crude form of the compound (Ia-4). This crude product was purified by means of a silica gel column chromatography using chloroform/methanol. Thus was obtained 13.64 g of the compound (Ia-4) (yield 20.0%). Melting point 75-78 °C.

Purity determined by gas chromatography: 97.8%

Hydroxyl value: 410.4 (theoretical value: 434.26)

IR (KBr, $cm^{-1}$):

3344 (O-H stretching, N-H stretching), 2932, 2864 (C-H stretching), 1628 (C=O stretching), 1532 (N-H deformation), 1468, 1384 (C-H deformation), 1058, 1026 (C-O deformation), 724 ($CH_2$ rocking), 676 (N-H out-of-plane deformation)

NMR ($CDCl_3$, δ ppm):

0.65 - 1.05 (6H, broad, -C<u>H</u>$_3$), 1.05 - 2.40 (29H, broad, -CH$_2$-,

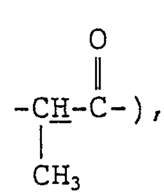

3.40 - 3.70 (6H, broad, -C<u>H</u>$_2$OH-), 4.75 - 5.60 (3H, broad, -CH$_2$O<u>H</u>), 6.40 - 6.55 (1H, broad,

$$-\overset{\displaystyle O}{\overset{\|}{C}}-N\underline{H}-)$$

EXAMPLE 18

Cleansing cream (composition 13 according to the invention):

| | (% by weight) |
|---|---|
| Sorbitol | 10 |
| Polyoxyethylene octyldodecyl ether (25 E.O.) | 15 |
| N-Tris(hydroxymethyl)methyl-α-methylstearamide (obtained in Example 15) | 60 |
| Glycerine | 5 |
| Dibutylhydroxytoluene | 0.1 |
| Methylparaben | 0.1 |
| Perfume | 0.1 |
| Water | Balance |
| Total | 100.0 |

The cleansing cream according to the invention was very stable and showed an excellent cleansing effect.

EXAMPLE 19

Cleansing cream (composition 14 according to the invention):

| | (% by weight) |
|---|---|
| Sorbitol | 10 |
| Polyoxyethylene octyldodecyl ether (25 E.O.) | 15 |
| N-Tris(hydroxymethyl)methyl-α-octylarachamide (obtained in Example 16) | 60 |
| Glycerine | 5 |
| Dibutylhydroxytoluene | 0.1 |
| Methylparaben | 0.1 |
| Perfume | 0.1 |
| Water | Balance |
| Total | 100.0 |

The cleansing cream according to the invention was very stable and showed an excellent cleansing effect.

EXAMPLE 20

Cleansing cream (composition 15 according to the invention):

| | (% by weight) |
|---|---|
| Sorbitol | 10 |
| Polyoxyethylene octyldodecyl ether (25 E.O.) | 15 |
| N-Tris(hydroxymethyl)methyl-α-heptylundecanamide (obtained in Example 17) | 60 |
| Glycerine | 5 |

| | |
|---|---|
| Dibutylhydroxytoluene | 0.1 |
| Methylparaben | 0.1 |
| Perfume | 0.1 |
| Water | Balance |
| Total | 100.0 |

The cleansing cream according to the invention was very stable and showed an excellent cleansing effect.

While the invention has been described in detail and with reference to specific embodiments thereof, it will be apparent to one skilled in the art that various changes and modifications can be made therein without departing from the spirit and scope thereof.

## Claims

1. An N-tris(hydroxymethyl)methyl-fatty acid amide of formula (I):

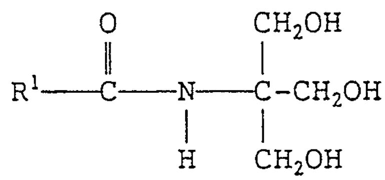

where $R^1$ represents a branched alkyl group containing 4 to 27 carbon atoms.

2. An N-tris(hydroxymethyl)methyl-fatty acid amide of Claim 1, wherein said N-tris(hydroxymethyl)methyl-fatty acid amide has formula (Ia):

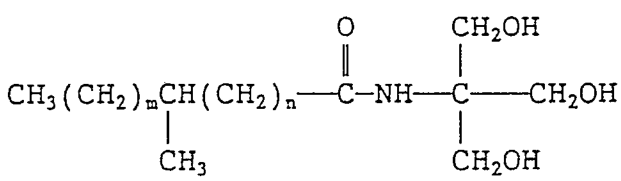

(Ia)

wherein m and n each, independently, represents an integer of 0 to 20 provided that the sum of m and n is 1 to 20.

3. An N-tris(hydroxymethyl)methyl-fatty acid amide of Claim 2, wherein m and n are each, independently, an integer of 0 to 16 provided that the sum of m and n is 10 to 16.

4. An N-tris(hydroxymethyl)methyl-fatty acid amide of Claim 2, wherein the sum of m and n is 14.

5. An N-tris(hydroxymethyl)methyl-fatty acid amide of claim 2, wherein m is 1 to 24 and n is 0.

6. An N-tris(hydroxymethyl)methyl-fatty acid amide of claim 1, wherein $R^1$ is the group represented by the formula

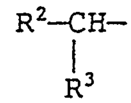

wherein $R^2$ and $R^3$ each represent a linear alkyl group containing 2 to 24 carbon atoms, provided that the sum of the carbon numbers of the alkyl groups represented by $R^2$ and $R^3$ is 4 to 26.

**7.** A method for producing N-tris(hydroxymethyl)methyl-fatty acid amide of Claim 1, which comprises the steps of:

reacting a branched fatty acid of formula (II):

$R_1COOH$ (II)

wherein $R_1$ is as defined above, with tris(hydroxymethyl)aminomethaneof formula (III):

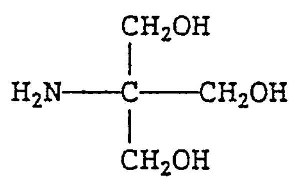

(III)

and hydrolyzing the resulting compound of formula (IV):

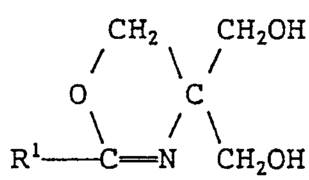

(IV)

wherein $R^1$ is as defined above, in a mixed solvent comprising a lower alcohol containing 1 to 6 carbon atoms and water.

**8.** A method of Claim 5, wherein the volume ratio of lower alcohol-to-water is in the range of 1:10 to 10:1.

**9.** A method of Claim 5, wherein said reacting step is conducted at a temperature of 100-250 °C.

**10.** A method of Claim 5, wherein said hydrolysing is conducted at a temperature of 50-150 °C

**11.** A cosmetic composition comprising an N-tris(hydroxymethyl)methyl-fatty acid amide of formula (I'):

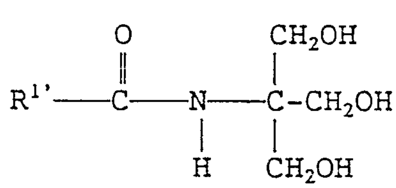

(I')

wherein $R^{1'}$ represents a branched alkyl or linear alkenyl group each containing 4 to 27 carbon atoms and a cosmetically acceptable base.

**12.** A cosmetic composition of Claim 11, wherein the N-tris(hydroxymethyl)methyl-fatty acid amide has

formula (Ia):

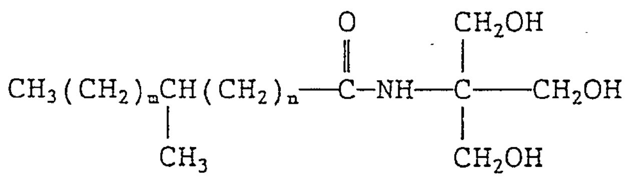

(Ia)

2

wherein m and n each, independently, represents an integer of 0 to 20 provided that the sum of m and n is 1 to 20.

13. A cosmetic composition of Claim 11, wherein m and n are each, independently, an integer of 0 to 16 provided that the sum of m and n is 10 to 16.

14. A cosmetic composition of Claim 11, wherein the sum of m and n is 14.

15. A cosmetic composition of claim 11, wherein the N-tris(hydroxymethyl)methyl-fatty acid amide has formula (Ia):

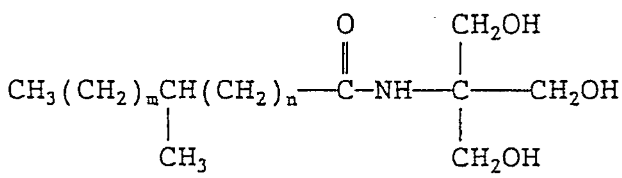

(Ia)

wherein m is 1 to 24 and n is 0.

16. A cosmetic composition of claim 11, wherein the N-tris(hydroxymethyl)methyl-fatty acid amide has formula (I):

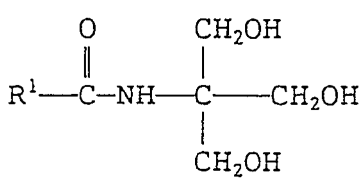

(I)

whose $R^1$ is represented by the formula

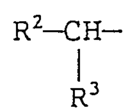

in which $R^2$ and carbon atoms, provided that the sum of the carbon numbers of the alkyl group represented by $R^2$ and $R^3$ is 4 to 26.

17. A method of moisturizing hair or skin, comprising contacting hair or skin with an effective amount of an N-tris(hydroxymethyl)methyl-fatty acid amide of formula (I'):

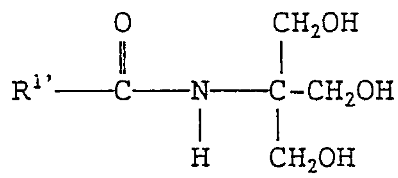

(I')

wherein $R^{1'}$ represents a branched alkyl or linear alkenyl group each containing 4 to 27 carbon atoms.

**18.** An oxazoline derivative of formula (IV):

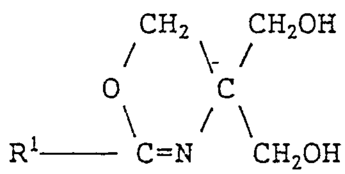

(IV)

wherein $R^1$ represents a group of the formula

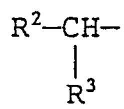

in which $R^2$ and $R^3$ each represents a linear alkyl group containing 2 to 24 carbon atoms, provided that the sum of the carbon numbers of the alkyl groups represented by $R^2$ and $R^3$ is 4 to 26.

Fig. 1

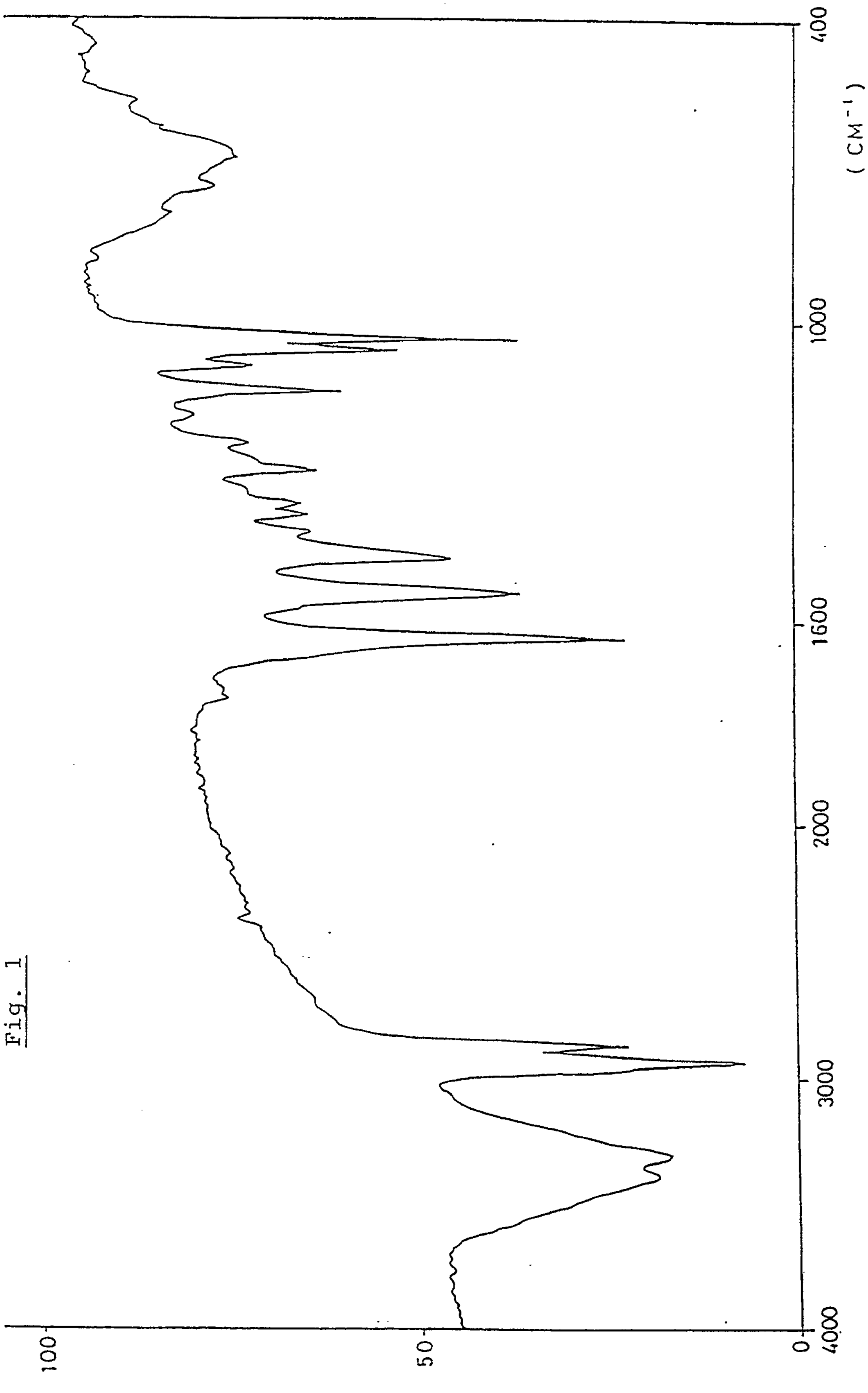

Fig. 2

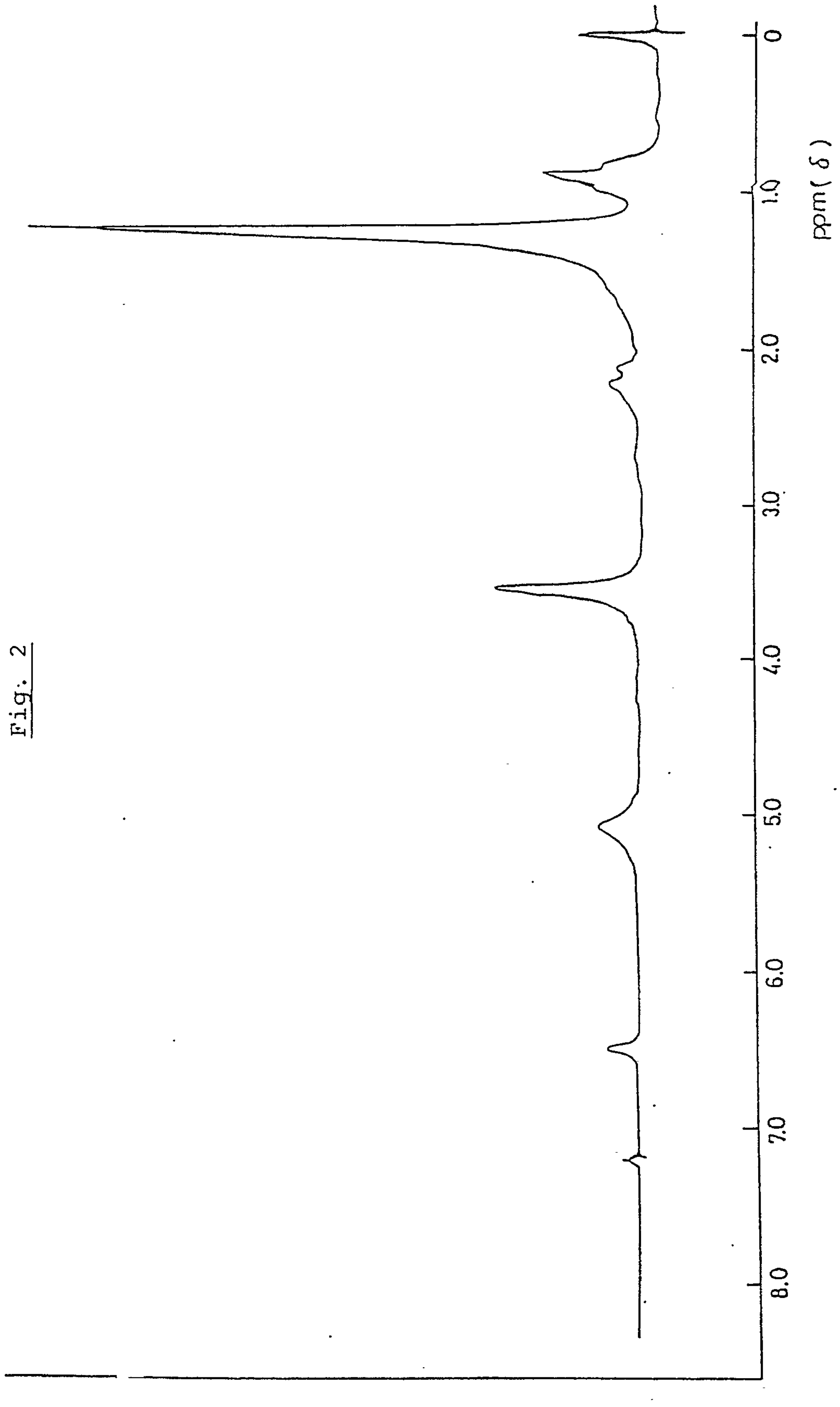

Fig. 3

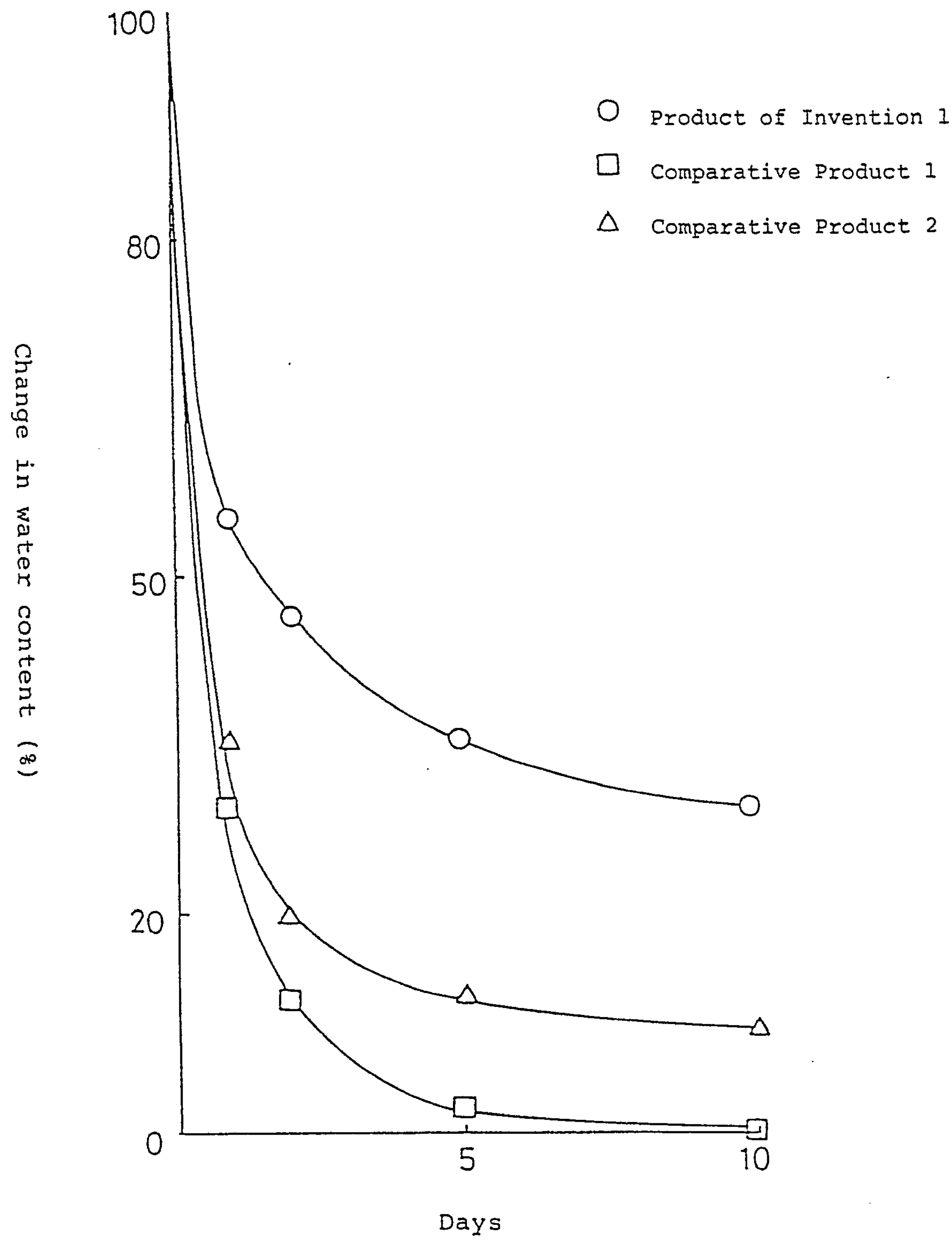
100
80
50
20
0
Change in water content (%)
5
10
Days
Product of Invention 1
Comparative Product 1
Comparative Product 2